(19) Europäisches Patentamt
European Patent Office
Office européen des brevets

(11) **EP 4 458 322 A1**

(12) **EUROPEAN PATENT APPLICATION**
published in accordance with Art. 153(4) EPC

(43) Date of publication:
**06.11.2024 Bulletin 2024/45**

(51) International Patent Classification (IPC):
***A61F 2/08*** *(2006.01)*

(21) Application number: **22914652.7**

(22) Date of filing: **26.12.2022**

(52) Cooperative Patent Classification (CPC):
**A61F 2/06; A61F 2/08; A61F 2/82; B05B 13/02;
B23D 79/00; B23K 26/38; B23K 26/70**

(86) International application number:
**PCT/CN2022/141879**

(87) International publication number:
**WO 2023/125398 (06.07.2023 Gazette 2023/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.12.2021 CN 202111682990**

(71) Applicant: **Biotyx Medical (Shenzhen) Co., Ltd.
Shenzhen, Guangdong 518000 (CN)**

(72) Inventors:
• **ZHANG, Wanqian
Shenzhen, Guangdong 518000 (CN)**
• **LI, Haifeng
Shenzhen, Guangdong 518000 (CN)**

(74) Representative: **Prinz & Partner mbB
Patent- und Rechtsanwälte
Rundfunkplatz 2
80335 München (DE)**

(54) **METHOD FOR KEEPING LONG TUBULAR IMPLANTABLE MEDICAL DEVICE STRAIGHT**

(57) The present invention belongs to the technical field of medical devices, and in particular, to a method for keeping a long-tubular implanted medical device flat and straight. The method is to fix one end of the long-tubular implanted medical device and apply an external force to the other end to ensure that the device is flat and straight. The method provided by the present invention is simple and convenient, and can ensure that an angle formed between the most inclined position of the long-tubular implanted medical device and the center line of a fixing device is $\theta \leq 5°$, thus improving the uniformity of a processed workpiece, the qualification rate of a processed product, and the detection accuracy.

FIG. 1

## Description

### Technical Field

The present invention belongs to the technical field of medical devices, and in particular to a method for keeping a long-tubular implanted medical device flat and straight.

### Background Art

With the development of medical technology, people have gradually paid attention to implanted medical devices. The implanted medical device mainly plays a supporting role in an affected area of a patient. For example, commonly used stent implantation in vascular diseases is to implant a stent into an affected area of a blood vessel, so that a constricted blood vessel can be reexpanded to restore normal blood supply, thereby achieving the purpose of revascularization. The commonly used stent implantation is currently the most effective way to treat diseases (such as coronary heart diseases and lower extremity atherosclerotic occlusive diseases) caused by vascular stenosis and insufficient blood supply. Due to the fact that the implanted medical device is implanted into the body of the patient, if the device has a low qualification rate, unstable quality, and the like, because of some factors in the production process, a large number of safety issues may be caused. This will cause very obvious side effects in the body of the patient and bring pain to the patient. Therefore, at present, all countries have set extremely high standards and strengthened corresponding supervision on the quality, safety, and other aspects of the implanted medical device. During production of products, manufacturers need to ensure that the products meet the corresponding standards and to avoid corresponding safety risks as much as possible after implantation.

In the production process of most medical devices, procedures such as spraying, cutting, and testing are involved. In the above procedures, a commonly used method is to clamp one end of the medical device with a clamp and suspend the other end, and then perform the corresponding operations such as spraying, cutting, and testing. If a medical device is relatively short, the suspended end will not bend and sag due to the gravity, so there are basically no problems of unstable device production process, low product qualification rate, and the like caused by the gravity during processing. When the length of a tubular medical device increases to a certain extent, especially for a long-tubular medical device with a hollow design, due to the low coverage of a surface material of the medical device, the entire device is light and thin. Therefore, a stent of the suspended part easily bends downwards under the action of the gravity. Especially in the procedures such as spraying, cutting, and testing, the device needs to rotate at a certain speed within a certain range, resulting in obvious "tail swinging" due to the non-overlapping of the central axes of front and rear ends of the device. Due to the rapid swinging of the suspended part, a series of problems occur in the production process. For example, a surface of the device cannot be sprayed uniformly in a spraying process; the energy is non-uniform during laser cutting, so that thorough cutting cannot be achieved, or the thickness of a cut rod is inconsistent; or, a non-uniform size, low processing accuracy, and the like are caused by an offset in a cut position. As a result, a series of problems such as a thrombus may occur when a finally produced stent product is used in the body. Based on this, it is necessary to provide a method for ensuring that a stent remains flat and straight throughout processing, which ensures that a coating layer on a surface of the stent is uniform, cut patterns are uniform, and the accuracy of a test result is high.

### Summary of the Invention

In response to the above-mentioned technical problems, the technical solution of the present invention provides a method for keeping a long-tubular implanted medical device flat and straight. This method can ensure that the long-tubular implanted medical device, especially a hollow long-tubular implanted medical device, is always maintained in a relatively flat and straight state in a manufacturing or testing procedure. The angle between a connection line and a straight line where the center line of a fixing device is located is $\theta \leq 5°$ and even $\theta \leq 3°$, and the connection line is between the highest point or lowest point through which the device passes during swinging and a fixed point of a fixed tail end of the device. Meanwhile, the qualification rate of an appearance and size of a workpiece in a manufacturing procedure and the testing accuracy in a testing procedure can reach 90% and even 95% and above.

The technical solution of the present invention provides a method for keeping a long-tubular implanted medical device flat and straight. The long-tubular implanted medical device in the method includes a fixed end and a suspended end, wherein an external force is applied to the suspended end to ensure that the device remains flat and straight.

The long-tubular implanted medical device in the present invention has a small specification. A longer tubular device has a longer suspended part, which will have a more serious tail swinging phenomenon. In some manufacturing procedures, since the processed device needs to rotate continuously, the device may have a significant tail swinging phenomenon due to the gravity and its own inertia. A tail swinging angle can reach 120°, which means that an angle formed by the connection line between the highest point through which the device passes during the swinging and the fixed point of the fixed tail end of the device and the connection between the lowest point through which the device passes during the swinging and the fixed point of the fixed tail end of the device is 2θ. A value of 2θ in the swinging process can be within 10° to 120°. Particularly when the long-tubular implanted medical device is of a hollow structure, the swinging is more serious. The swinging angle 2θ can even reach 180°. Therefore, a sufficient force needs to be applied to a surface of the device to achieve an effect of "pulling a tail part of the device straight".

It should be noted that the above "highest point" and "lowest point" both refer to the center point of a cross section of a tail end of the device at the highest or lowest position where the device reaches during the swinging.

In the above-mentioned technical solution provided by the present invention, the external force provided is a traction force of a magnetic field. The magnitude of the magnetic traction force acting on the implanted medical device is

$$F_{pull} = \int_{x}^{x+h} \frac{B^2 S}{2 \times \mu_0} dx \quad \text{Formula I}$$

where $\mu_0$ is the magnetic permeability (namely, a vacuum magnetic permeability, where $\mu_0 = 4\pi \times 10^{-7} \text{N·A}^{-2}$) of a magnetic medium in a vacuum state; x is the distance from a magnetic source to the most epitaxial acting surface of a magnetically conductive material in the suspended end of the device; dx is the integral of the distance from the magnetic source to the acting surface of the magnetically conductive material; h is the length of the magnetically conductive material on the device in a direction perpendicular to the acting surface; $F_{pull}$ is the total traction force the magnetic field on the device; B is the magnetic induction intensity of the magnetic field at the acting surface of the magnetically conductive material; and S is the area of the magnetic field on the acting surface of the magnetically conductive material.

The long-tubular implanted medical device in the present invention can generate a corresponding acting force under the induction of the magnetic field, but the specific way for applying the magnetic traction force to the surface of the device is not limited. In some cases, the long-tubular implanted medical device itself has magnetic conductivity. For example, in some embodiments, the entire suspended part or even the entire device can be magnetically conductive and subjected to the acting force of the magnetic field. In some other embodiments, a certain part of the device has the magnetic conductivity. For example, a coating layer of the device or a main material of the device has the magnetic conductivity, or there is a substance (such as a developing structure) with the magnetic conductivity on a certain cross section of the device, so that the device undergoes the acting force of the magnetic field, thereby ensuring that the device can be kept in a flat and straight state. In some other cases, the device itself is not magnetically conductive. However, by virtue of some external materials with magnetic conductivity, the device can also remain flat and straight under the acting force of the magnetic field. For example, some magnetically conductive patches are mounted on an outer surface of the device.

When the device itself has the magnetic conductivity and its suspended part or the entire device is magnetically conductive, if the device is made of pure iron or a ferroalloy material, the acting force of the magnetic field on the device in the present invention is $F_{pull} = \int_{x}^{x+h} \frac{B^2 S}{2 \times \mu_0} dx$ , where $\mu_0$ is a magnetic permeability of a magnetic medium in a vacuum state, namely, a vacuum magnetic permeability, where $\mu_0 = 4\pi \times 10^{-7} \text{N·A}^{-2}$; x is the distance from a magnetic source to the most epitaxial acting surface in the suspended end of the device; dx is the integral of the distance from the magnetic source to the acting surface of the device; h is the length of the suspended part of the device; $F_{pull}$ is the total magnetic traction force on the device; B is the magnetic induction intensity of the magnetic field at the acting surface of the device; and S is the area of the magnetic field on the acting surface of the device.

In the present invention, the "area S of the magnetic field on the acting surface of the device" refers to the area of a covering material on any circumferential cross section of the device under the action of the magnetic force. When the device in the present invention is of a hollow tubular structure, the "area S of the magnetic field on the acting surface of the device" refers to the area of a device supporting rod on a cross section, namely, $S_{\text{rod section}}$. A calculation formula of the area is a circumferential cross-sectional area $S_{\text{circumferential}}$ of the outer diameter of the device multiplied by the coverage $S_{\text{section coverage}}$ of a material covered by the cross section of the device, which is:

$$S_{\text{rod section}} = S_{\text{circumferential}} \times S_{\text{section coverage}} \qquad \text{Formula II}$$

where the coverage $S_{\text{section coverage}}$ of the material covered by the device on the cross section refers to the ratio of the area $S_{\text{rod section}}$ of the supporting rod on the entire circumferential cross section to the circumferential cross sectional area $S_{\text{circumferential}}$ of the outer diameter of a stent, that is:

$$S_{\text{section coverage}} = \frac{S_{rod\ section}}{S_{circumferential}} \times 100\% \qquad \text{Formula III}$$

It should be noted that the $S_{\text{section coverage}}$, $S_{\text{circumferential}}$, and $S_{\text{rod section}}$ mentioned above are all average values and represent areas of the device in a processing state/process, instead of areas of the device after the device is implanted in the body and in use.

When the device itself in the present invention is only partially magnetically conductive (internally magnetically conductive) or has a magnetically conductive material (externally magnetically conductive) attached to its outer surface, especially when the magnetically conductive material at a magnetically conductive position inside or outside the device is extremely short in a lengthwise direction of the device and can be even ignored, the formula of the above traction force may be:

$$F_{pull} = \frac{B^2 S}{2 \times \mu_0} \qquad \text{Formula IV,}$$

that is, the magnitude of the magnetic traction force on the device is only related to an area of the magnetically conductive material in a circumferential cross-sectional direction.

Due to the gradual attenuation of the magnetic field with an increasing distance, in a constant magnetic field emitted by a certain magnetic source, the magnetic induction intensity of the magnetic field is lower if the magnetic source is farther. Therefore, when the distance between the magnetic field and the most epitaxial acting surface of the suspended end of the implanted medical device is longer, in order to ensure that the final force acting on the surface of the device remains constant, it is necessary to correspondingly increase the magnetic induction intensity emitted by the magnetic source. When the distance between the magnetic source and the most epitaxial acting surface of the suspended end of the implanted medical device is long, the desired magnetic induction intensity can reach 1000 mT. When the distance between the magnetic source and the most epitaxial acting surface of the suspended end of the implanted medical device is short, the magnetic induction intensity generated by the magnetic source is 100 mT, which can also meet the requirement. In the above-mentioned technical solution provided by the present invention, the magnetic induction intensity of the magnetic field generated by the magnetic source is B ≤ 1000 mT, including but not limited to any value below 1000, such as 200 mT, 300 mT, 400 mT, 500 mT, 600 mT, 700 mT, 800 mT, and 900 mT. Further, the magnetic induction intensity of the magnetic field generated by the magnetic source is B ≤ 750 mT or B ≤ 600 mT.

In the above-mentioned technical solution provided by the present invention, the magnetic source that generates the magnetic traction force can be any device and/or equipment that can generate a magnetic field, which can be naturally magnetic, such as a commonly used permanent magnet, or a device or equipment that can generate a magnetic field under the action of an external force, such as a device or equipment that can generate a magnetic field when powered on. Further, the magnetic source of the present invention includes but is not limited to a permanent magnet and an electromagnet, which can be a magnetic stone, a magnet, a coil that generates a magnetic field, and the like. The magnetic source can also be any one or more of a constant magnetic field, an alternating magnetic field, a pulsating magnetic field, or a pulsed magnetic field. In addition, a quantity of the magnetic field source is not limited to one. The quantity can be one or multiple.

In the above-mentioned technical solution provided by the present invention, the value range of the distance x between the magnetic source and the most epitaxial acting surface of the suspended end of the implanted medical device is 0.1 mm to 100 mm (including but not limited to 0.5 mm, 1 mm, 3 mm, 5 mm, 7 mm, 9 mm, 15 mm, 20 mm, 25 mm, 30 mm, 35 mm, 40 mm, 45 mm, 50 mm, 55 mm, 60 mm, 65 mm, 70 mm, 75 mm, 80 mm, 85 mm, 90 mm, 95 mm, 98 mm, and the like). Further, the value of the distance x between the magnetic source and the most epitaxial acting surface of the suspended end of the implanted medical device can be in a range composed of any two values between 0.1 mm to 100 mm, such as 0.1 mm to 10 mm, 0.1 mm to 15 mm, 0.1 mm to 25 mm, 0.1 mm to 50 mm, 0.1 mm to 60 mm, 0.1 mm to 70 mm, 0.1 mm to 80 mm, 0.15 mm to 80 mm, and 0.15 mm to 90 mm.

The most epitaxial acting surface of the suspended end of the implanted medical device in the present invention refers to a circumferential cross section of a point, closest to the magnetic source, on the magnetically conductive material in a direction perpendicular to a radial surface of the device. When the entire device or the suspended part of the device in the present invention is made of the magnetically conductive material, the most epitaxial acting surface of the suspended end of the implanted medical device refers to a circumferential cross section, which is in contact with air and is perpendicular to the radial surface, of the suspended end of the device.

The present invention is a technical solution specially designed for the manufacturing procedure of the long-tubular implanted medical device. If the implanted medical device is longer, the surface coverage is lower. Therefore, the implanted medical device is more prone to bending and tail swinging during rotation or vibration due to the gravity.

However, since a medical device required/applicable to the body has a limited length, the present invention is applicable to keeping a long-tubular device with a length in a suspended part of 5 mm to 200 mm (including but not limited to: the length of the suspended part being 10 mm, 15 mm, 20 mm, 25 mm, 30 mm, 35 mm, 40 mm, 45 mm, 50 mm, 55 mm, 60 mm, 65 mm, 70 mm, 75 mm, 80 mm, 85 mm, 90 mm, 95 mm, 100 mm, 110 mm, 120 mm, 130 mm, 140 mm, 150 mm, 160 mm, 170 mm, 180 mm, 190 mm, and 200 mm) flat and straight, and is further applicable to a long-tubular device with a length in a suspended part of a new range composed of any two values within 5 mm to 200 mm, such as 38 mm to 150 mm, 38 mm to 140 mm, 15 mm to 150 mm, 15 mm to 140 mm, 20 mm to 150 mm, and 20 mm to 140 mm.

In the above-mentioned technical solution provided by the present invention, in order to ensure that the device is basically kept flat and straight, that is, to ensure that an angle $\theta$ formed between the most inclined position of the device and the center line of a fixing device is very small, the value of the angle $\theta$ can be controlled at 5° and below 5°, and can even reach 3° and below 3°, thus ensuring that the qualification rate of an appearance and size of a workpiece in a manufacturing procedure and the testing accuracy in a testing procedure can reach 90% or above. Therefore, a sufficient external force or magnetic traction force needs be applied to the suspended end of the stent. However, the magnetic traction force applied to the implanted medical device should not be too high. A traction force that is too high can easily cause the device to break away from the fixed end and lose stability, that is, the magnitude of the magnetic traction force acting on the implanted medical device needs to be less than the tension of the fixed end on the device, which ensures that the device at the fixed end will not move left and right or up and down. In addition, when the long-tubular implanted medical device in the present invention is of a hollow structure, if the applied external force is too high, the device is easily pulled to deform. Therefore, in the present invention, the magnitude of the magnetic traction force applied to the implanted medical device needs to be limited to ensure that the magnitude of the magnetic traction force acting on the device is appropriate. In the technical solution provided by the present invention, the magnitude of the magnetic traction force acting on the device is 1 to 40 times the gravity of the implanted medical device, including but not limited to twice, 3 times, 5 times, 7 times, 10 times, 12 times, 15 times, 18 times, 20 times, 23 times, 25 times, 28 times, 30 times, 35 times, 38 times, 40 times, and the like. Further, the magnitude of the magnetic traction force acting on the implanted medical device is 1 to 28 times, 1 to 20 times, 5 to 35 times, 3 to 35 times, and 3 to 40 times the gravity of the implanted medical device.

In a spraying procedure of the long-tubular implanted medical device, spraying liquid is very viscous. If the spraying liquid is sprayed onto the end of the device that has a clamp during spraying, it can easily cause the device to adhere to the clamp. As a result, a coating layer on the surface of the device is torn during separation from the clamp. Therefore, in a common way, the device is sprayed two or more times, which also requires that the length of the clamp at a clamping end of the implanted medical device may not exceed the length of the device, that is, the length of the device needs to be greater than the length of the clamp. In the above technical solution provided by the present invention, the length of the clamp is less than or equal to $\frac{2}{3}$ of the length of the implanted medical device, or is less than or equal to $\frac{1}{2}, \frac{1}{3}, \frac{1}{4}$, and $\frac{1}{5}$ of the length of the implanted medical device, or the length of the clamp located inside the device may also be 0 mm.

In the above technical solution provided by the present invention, the fixation method for the fixed end of the implanted medical device is diverse. The fixed end may be clamped by a clamp, or may be fixed by physical interference fit, or may be fixed magnetically or chemically. That is, the fixation method for the fixed end of the device in the technical solution provided by the present invention includes but is not limited to clamping, physical interference fit, magnetic fixation, and chemical fixation.

The above preparation method provided by the present invention is suitable for processing of long-tubular implanted medical devices with all outer diameters, wall thicknesses, and coverages, and is especially suitable for processing of hollow long-tubular implanted medical devices. It ensures that the long-tubular implanted medical device remains flat and straight during the processing.

In the above technical solution provided by the present invention, the outer diameter of the long-tubular implanted medical device is 1.0 mm to 20.0 mm. Further, the outer diameter of the long-tubular implanted medical device is 1.0 mm to 15.0 mm. Furthermore, the outer diameter of the long-tubular implanted medical device is 1.0 mm to 10.0 mm.

In the above technical solution provided by the present invention, the wall thickness of the long-tubular implanted medical device is 10 $\mu$m to 600 $\mu$m (including but not limited to 20 $\mu$m, 30 $\mu$m, 40 $\mu$m, 50 $\mu$m, 80 $\mu$m, 100 $\mu$m, 120 $\mu$m, 150 $\mu$m, 175 $\mu$m, 200 $\mu$m, 225 $\mu$m, 250 $\mu$m, 280 $\mu$m, 300 $\mu$m, 320 $\mu$m, 350 $\mu$m, 380 $\mu$m, 400 $\mu$m, 425 $\mu$m, 450 $\mu$m, 475 $\mu$m, 500 $\mu$m, 520 $\mu$m, 550 $\mu$m, 560 $\mu$m, 580 $\mu$m, 600 $\mu$m, and the like). Further, the wall thickness of the long-tubular implanted medical device is 10 $\mu$m to 500 $\mu$m. Furthermore, the wall thickness of the long-tubular implanted medical device is 15 $\mu$m to 450 $\mu$m.

In the above technical solution provided by the present invention, the coverage of the cross section of the long-tubular implanted medical device is 0.1% to 35% (including but not limited to 1%, 5%, 8%, 10%, 12%, 14%, 16%, 18%, 20%, 22.5%, 25%, 28%, 30%, 33%, 34.5%, and the like). Further, the coverage of the cross section of the long-tubular implanted medical device is 0.1% to 30%. Furthermore, the coverage of the cross section of the long-tubular implanted medical device is 0.1% to 25%.

In the above technical solution provided by the present invention, the long-tubular implanted medical device is of hollow design, with a surface coverage of 5% to 60% (including but not limited to 6%, 8%, 10%, 12%, 14%, 16%, 18%, 20%, 22.5%, 25%, 28%, 30%, 33%, 36%, 40%, 45%, 50%, 55%, 58%, and the like). Further, the coverage of the long-tubular implanted medical device is 5% to 55%. Furthermore, the coverage of the long-tubular implanted medical device is within the range of any two values forming an interval within 5% to 60%, such as 5% to 50%, 8% to 55%, 8% to 50%, 10% to 55%, 10% to 50%, 8% to 45%, 8% to 40%, 5% to 45%, 5% to 40%, 10% to 45%, 10% to 40%, 8% to 35%, or 10% to 35%.

It should be noted that the "surface coverage of the device" in the present invention refers to a surface coverage of a device material under a device diameter condition during the manufacturing process, that is, the ratio of a surface area covered by the device material to a total cylindrical side area of a device covering section. A formula is as follows:

$$\text{Surface coverage} = A = \frac{S_r}{S_s} \times 100\% \qquad (1),$$

where

Sr: an actual filled/occupied area on an outer surface of a device pattern during the manufacturing process, where an outer surface area of the stent is measured using CAD software;

Ss: the total cylindrical side area of the stent covering section during the manufacturing process, $Ss=\pi\times D1\times L1$ (2)

D1: the diameter of the stent during the manufacturing process.

It should be noted that "outer diameter", "wall thickness", "cross sectional coverage", and "surface coverage" of the long-tubular implanted medical device in the present invention all refer to corresponding values of the long-tubular implanted medical device in a processing state, namely, values of "outer diameter", "wall thickness", "cross sectional coverage", and "surface coverage" of a tube material that is to be processed or being processed into the long-tubular implanted medical device.

In the technical solution provided by the present invention, in some other embodiments, the long-tubular implanted medical device is a lumen prosthesis. Further, the long-tubular implanted medical device is a blood flow guide device. Furthermore, the long-tubular implanted medical device is a vascular supporter. In some embodiments of the present invention, the long-tubular implanted medical device is a stent. In some other embodiments, the long-tubular implanted medical device is a tube material to be processed into a stent. In some embodiments, the long-tubular implanted medical device is a semi-finished product to be processed into a finished stent product. In some other embodiments, the long-tubular implanted medical device can be any implantable long and lightweight medical device used in the body. In still some other embodiments, the long-tubular implanted medical device is a raw material or semi-finished product to be processed into a stent, or a finished stent product.

In the above technical solution provided by the present invention, the implanted medical device itself has magnetic conductivity. Or, a magnetic patch is added onto the implanted medical device. The magnetic patch can be mounted at any position on the device. However, the effect is better if the magnetic patch is mounted at a position that is not clamped by a clamp and is closer to the magnetic source, including but not limited to adding magnetically conductive development points at two ends of the stent, or applying a magnetically conductive coating layer to the surface of the stent. Further, it is better if the average distance between the magnetic patch and the magnetic source is shorter. There may be one or more magnetic patches in the present invention. The shape of the magnetic patch is not limited, as long as the magnetic path can generate a sufficient magnetic force in conjunction with the intensity of the magnetic field generated by the magnetic source and the distance between the acting surface of the device and the magnetic source to keep the stent flat and straight.

This method is applicable to a plurality of procedures in the production process of the hollow long-tubular device, including but not limited to spraying, cutting, testing, lamination, and the like.

In the above technical solutions provided by the present invention, in some embodiments, a material of the device may be pure iron or iron-based alloy, or any other pure metal or alloy material with magnetic conductivity. In some other embodiments, the material of the device may be a substance such as polylactic acid that does not have magnetic conductivity.

The "fixing" in the present invention can be achieved by fixing the fixed end of the device at a specific position through a force generated by an interaction between the clamp and the device, or can be achieved by fixing the fixed end of the device at a specific position through an external force such as a magnetic force, or can be achieved through interference fit or through a chemical action of the magnetic field.

It should be noted that in order to facilitate the description, "long-tubular implanted medical device" has been simplified, that is, "device", "medical device", and "implanted medical device" in the present invention all refer to "long-tubular implanted medical device".

The "long-tubular" implanted medical device in the present invention refers to a device having a length greater than an outer diameter, especially a device with a length in a suspended part greater than or equal to 5 mm, and further a device with a length in a suspended part greater than 10 mm.

The term "tail swinging" in the present invention refers to a central axis of a front end of the stentand a central axis of a rear end of the stent not overlapping during the manufacturing process.

The "flat and straight" in the present invention refers to the angle formed between the most inclined position of the implanted medical device and the center line of the fixing device being $\theta \le 5°$.

It should be noted that symbol "/" in the present invention represents the meaning of "or". For example, "required/applicable to the body" refers to being required or applicable to the body.

It should be understood that the terms used herein are only for the purpose of describing specific exemplary implementations and are not intended to be restrictive. Unless otherwise explicitly stated in the context, the singular forms such as "a/an", "one", and "the" used herein can also indicate a plural form. The terms "include", "contain", "containing", and "have" are inclusive and therefore indicate the existence of the stated features, steps, operations, elements, and/or components, but do not exclude the existence or addition of one or more other features, steps, operations, elements, components, and/or their combinations. The method steps, processes, and operations described herein are not interpreted as requiring them to be executed in a specific order described or explained, unless an order of execution is clearly indicated. It should also be understood that other or alternative steps can be used.

## Brief Description of the Drawings

Various other advantages and benefits will become apparent to those of ordinary skill in the art upon reading the following detailed description of the preferred implementations. The accompanying drawings are only for the purpose of illustrating preferred implementations and are not considered as a limitation on the present invention. Furthermore, the same components are denoted by the same reference numerals throughout the drawings. Where:

FIG. 1 is a schematic diagram of a spraying state of a stent in Embodiment 1;

FIG. 2 is a schematic diagram of a combination method for a developing hole and a stent in Embodiment 5; and

FIG. 3 is a schematic diagram of a state of a cutting process of a stent in Embodiment 6.

## Detailed Description of the Invention

The exemplary implementations of the present invention will be described in more detail below with reference to the accompanying drawings. Although the accompanying drawings show the exemplary implementations of the present invention, it should be understood that the present invention can be implemented in various forms, and should not be limited to the implementations stated herein. Rather, these implementations are provided for understanding the present invention more thoroughly, and can completely transfer the scope of the present invention to those skilled in the art.

Test method:

1. Test on thicknesses of a coating layer

A stent is placed under an ultrasonic atomization nozzle and is sprayed. After a layer of coating covers a surface of the stent, a sensofar-non-contact 3d optical profilometer is used to measure the thicknesses of the coating layer on the surface of the stent based on the principle of white light interferometry. The thicknesses of the coating layer at multiple positions of the entire stent are measured and are compared to obtain a maximum value and a minimum value from the thicknesses of the coating layer. The difference value between the maximum value and the minimum value should not exceed 2 $\mu$m.

### 2. Test on widths of stent rods

Cut stents are placed under a three-dimensional microscope for observation. All stent rods are traversed with the eyes to find a rod with a maximum rod width and a rod with a minimum rod width for measurement. If the difference between the maximum rod width and the minimum rod width is measured to exceed 16 $\mu$m, the two rods are defined to be male and female rods. The quantity of male and female stent rods obtained by cutting is counted. There may be:

Qualification rate of cutting size = quantity of male and female stent/quantity of cut stents.

### 3. Measurement of an inclination angle

The length h of a suspended section of the stent is determined. The height b from the center point of the farthest end cross section of the stent to the horizontal center line of a clamping section is measured. According to a trigonometric function, there may be $\theta = \arcsin\dfrac{b}{h}$ , thus obtaining the size of the inclination angle $\theta$.

### 4. Test on a cut appearance

The cut stents are placed under the three-dimensional microscope to observe whether surfaces of the stents have surplus material. The quantity of stents that are with surplus material is counted.

There may be: qualification rate of cutting appearance = quantity of stents that are with surplus material/quantity of cut stents.

### Embodiment 1

One end of an iron-based absorbable drug elution peripheral stent with a length of 118 mm was fixed by a clamp with a length of 59 mm through physical interference fit (the processing outer diameter of the stent was 1.58 mm; the surface coverage of the stent was 30%; the wall thickness of the stent was 70 $\mu$m; the coverage of supporting rods on a circumferential cross section of the stent was 5%; and the mass m of the stent was 112.53 mg). The length h of a clamping-free section was 59 mm. A magnet having a maximum magnetic induction intensity $B_{max}$ of 400 mT was used as a magnetic source to apply a magnetic traction force to the stent. When the magnet was arranged at a position that was 1 mm away from the most epitaxial acting surface of a suspended end of the stent, the stent might be flat and straight. An exponential function curve of a magnetic induction intensity and a distance x was fitted according to magnetic induction intensities B of the magnet measured at different positions through a gauss meter. The exponential function curve was substituted into the formula for $F_{pull}$, it could be calculated that $F_{pull}$=0.00844 N, and the gravity of the suspended part of the stent was $F_{gravity}$=0.00056 N. Therefore, $F_{pull} \approx 15F_{gravity}$. At this time, the height from the center point of the farthest end cross section of the stent and the horizontal center line of a clamping section was b=3.09 mm. It was calculated according to the trigonometric function that $\theta$=3°<5°. The stent in the above state was placed under an ultrasonic atomization nozzle for spraying. A sensofar-non-contact 3D optical profilometer was used to measure coating thicknesses of the sprayed stent, and the difference between the maximum coating thickness and the minimum coating thickness was 0.5 $\mu$m.

### Embodiment 2

One end of an iron-based absorbable drug elution peripheral stent with a length of 200mm was fixed by a clamp with a length of 100mm through physical interference fit (the processing outer diameter of the stent was 1.58 mm; the surface coverage of the stent was 30%; the wall thickness of the stent was 60 $\mu$m; the coverage of supporting rods on a circumferential cross section of the stent was 4%; and the mass m of the stent was 152.16 mg). The length h of a clamping-free section was 100mm. A magnet having a maximum magnetic induction intensity $B_{max}$ of 600 mT was used as a magnetic source to apply a magnetic traction force to the stent. When the magnet was arranged at a position that was 1 mm away from the most epitaxial acting surface of a suspended end of the stent, the stent might be flat and straight. An exponential function curve of a magnetic induction intensity and a distance x was fitted according to magnetic induction intensities B of the magnet measured at different positions through a gauss meter. The exponential function curve was substituted into the formula for $F_{pull}$, it could be calculated that $F_{pull}$ =0.01902N, and the gravity of the suspended part of the stent was $F_{gravity}$ = 0.00076N. Therefore, $F_{pull} \approx 25F_{gravity}$. At this time, the height from the center point of the farthest end cross section of the stent to a horizontal center line of a clamping section was b=6.98 mm. It was calculated according to the trigonometric function that $\theta$=4°<5°. The stent in the above state was placed under an ultrasonic atomization nozzle for spraying. A sensofar-non-contact 3D optical profilometer was used to measure coating thicknesses of the sprayed stent, and the difference between the maximum coating thickness and the minimum coating thickness was 1 $\mu$m.

Embodiment 3

One end of an iron-based absorbable drug elution peripheral stent with a length of 38 mm was fixed by a clamp with a length of 15 mm through physical interference fit (the processing outer diameter of the stent was 1.2 mm; the surface coverage of the stent was 40%; the wall thickness of the stent was 80 $\mu$m; the coverage of supporting rods on a circumferential cross section of the stent was 10%; and the mass m of the stent was 22.83 mg). The length h of a clamping-free section was 23 mm. A magnet having a maximum magnetic induction intensity $B_{max}$ of 300 mT was used as a magnetic source to apply a magnetic traction force to the stent. When the magnet was arranged at a position that was 3 mm away from the most epitaxial acting surface of a suspended end of the stent, the stent might be flat and straight. An exponential function curve of a magnetic induction intensity and a distance x was fitted according to magnetic induction intensities B of the magnet measured at different positions through a gauss meter. The exponential function curve was substituted into the formula for $F_{pull}$, it could be calculated that $F_{pull}$ =0.00209N, and the gravity of the suspended part of the stent was $F_{gravity}$= 0.00026N. Therefore, $F_{pull} \approx 8F_{gravity}$. At this time, the height from the center point of the farthest end cross section of the stent to the horizontal center line of a clamping section was b=2.00 mm. It was calculated according to the trigonometric function that $\theta$=5°. The stent in the above state was placed under an ultrasonic atomization nozzle for spraying. A sensofar-non-contact 3D optical profilometer was used to measure coating thicknesses of the sprayed stent, and the difference between the maximum coating thickness and the minimum coating thickness was 1 $\mu$m.

Embodiment 4

One end of a cobalt alloy stent with a length of 118 mm was fixed by a clamp with a length of 50 mm through physical interference fit (the processing outer diameter of the stent was 2.4 mm; the surface coverage of the stent was 20%; the wall thickness of the stent was 100 $\mu$m; the coverage of supporting rods on a circumferential cross section of the stent was 4%; and the mass m of the stent was 98.8 mg). The length h of a clamping-free section was 68 mm. A magnet having a maximum magnetic induction intensity $B_{max}$ of 300 mT was used as a magnetic source to apply a magnetic traction force to the stent. When the magnet was arranged at a position that was 1 mm away from the most epitaxial acting surface of a suspended end of the stent, the stent might be flat and straight. An exponential function curve of a magnetic induction intensity and a distance x was fitted according to magnetic induction intensities B of the magnet measured at different positions through a gauss meter. The exponential function curve was substituted into the formula for $F_{pull}$, it could be calculated that $F_{pull}$ =0.00878 N, and the gravity of the suspended part of the stent was $F_{gravity}$=0.00057 N. Therefore, $F_{pull} \approx 15F_{gravity}$. At this time, the height from a center point of the farthest end cross section of the stent to the horizontal center line of a clamping section was b=4.74 mm. It was calculated according to the trigonometric function that $\theta$=4°<5°. The stent in the above state was placed under an ultrasonic atomization nozzle for spraying. A sensofar-non-contact 3D optical profilometer was used to measure coating thicknesses of the sprayed stent, and the difference between the maximum coating thickness and the minimum coating thickness was 0.5 $\mu$m.

Embodiment 5

One end of an iron-based absorbable drug elution stent with a length of 58 mm was fixed by a clamp with a length of 15 mm through physical interference fit (the processing outer diameter of the stent was 8.0 mm; the surface coverage of the stent was 11%; the wall thickness of the stent was 150 $\mu$m; the coverage of supporting rods on a circumferential cross section of the stent was 0.8%; and the mass m of the stent was 215.98 mg). The length h of a clamping-free section was 43 mm. A magnet having a maximum magnetic induction intensity $B_{max}$ of 600 mT was used as a magnetic source to apply a magnetic traction force to the stent. When the magnet was arranged at a position that was 3 mm away from the most epitaxial acting surface of a suspended end of the stent, the stent might be flat and straight. An exponential function curve of a magnetic induction intensity and a distance x was fitted according to magnetic induction intensities B of the magnet measured at different positions through a gauss meter. The exponential function curve was substituted into the formula for $F_{pull}$, it could be calculated that $F_{pull}$ =0.04937 N, and the gravity of the suspended part of the stent was $F_{gravity}$=0.00160 N. Therefore, $F_{pull} \approx 31F_{gravity}$. At this time, the height from the center point of the farthest end cross section of the stent to the horizontal center line of a clamping section was b=3.75 mm. It was calculated according to the trigonometric function that $\theta$=5°. The stent in the above state was placed under an ultrasonic atomization nozzle for spraying. A sensofar-non-contact 3D optical profilometer was used to measure coating thicknesses of the sprayed stent, and the difference between the maximum coating thickness and the minimum coating thickness was 1 $\mu$m.

Embodiment 6

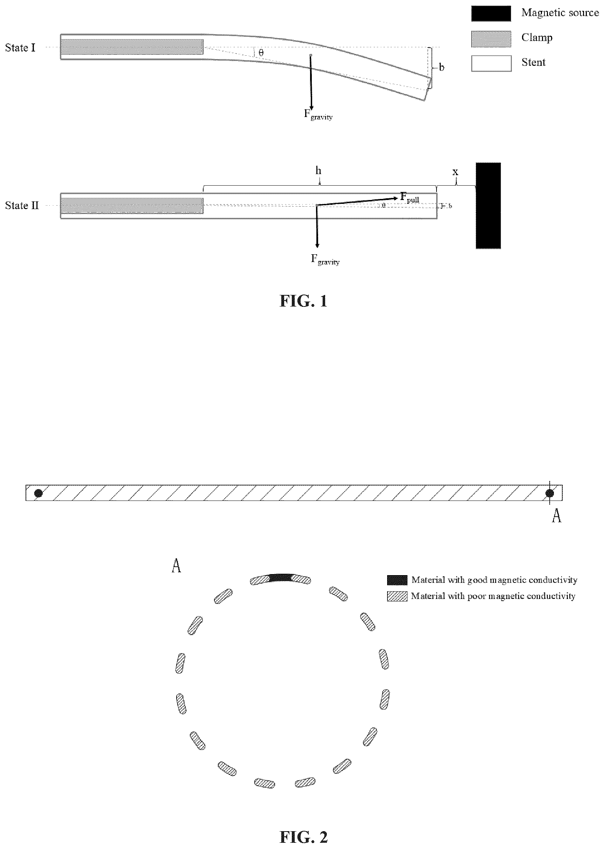

Each of two ends of a magnesium alloy stent (the mass m of which was 57.26 mg) with a length of 68 mm was cut to form a developing hole with a cross sectional area of 0.5 mm$^2$. The developing holes were filled with a nickel material with good magnetic conductivity (as shown in FIG. 2). The clamp used had a length of 30 mm, and the length h of a clamping-free section of the stent was 38 mm. A magnet having a maximum magnetic induction intensity $B_{max}$ of 300 mT was used as a magnetic source to apply a magnetic traction force to the stent. When the magnet was arranged at a position that was 3 mm away from the developing holes, the stent might be flat and straight. Since the stent undergone the traction force only at a developing point, and the length h of the developing point in a radial direction of the stent was too small, the traction force on the stent was calculated according to the formula $F_{pull} = \dfrac{B^2 S}{2 \times \mu_0}$. It was calculated that $F_{pull}$=0.00447 N according to the magnetic induction intensity B=150 mT, measured by a gauss meter, of the magnet in case of x=3. The gravity on the suspended part of the stent was $F_{gravity}$=0.00032 N. In this case, $F_{pull}$=14$F_{gravity}$. At this time, the height from the center point of the farthest end cross section of the stent and the horizontal center line of the clamping section was b=2.65 mm. It was calculated according to the trigonometric function that $\theta = 4° < 5°$. The stent in the above state was placed under an ultrasonic atomization nozzle for spraying. A sensofar-non-contact 3D optical profilometer was used to measure coating thicknesses of the sprayed stent, and the difference between the maximum coating thickness and the minimum coating thickness was 0.5 $\mu$m.

Embodiment 7

One end of an iron-based absorbable drug elution peripheral stent with a length of 38 mm was fixed by a magnet through magnetic suction (the processing outer diameter of the stent was 1.58 mm; the surface coverage of the stent was 30%; the wall thickness of the stent was 70 $\mu$m; the coverage of supporting rods on a circumferential cross section of the stent was 5%; and the mass m of the stent was 36.24 mg). The length h of the clamp without a clamping section was 38mm. A magnet having a maximum magnetic induction intensity $B_{max}$ of 400 mT was used as a magnetic source to apply a magnetic traction force to the stent. When the magnet was arranged at a position that was 1 mm away from the most epitaxial acting surface of a suspended end of the stent, the stent might be flat and straight. An exponential function curve of a magnetic induction intensity and a distance x was fitted according to magnetic induction intensities B of the magnet measured at different positions through a gauss meter. The exponential function curve was substituted into the formula for $F_{pull}$, it could be calculated that $F_{pull}$=0.00844 N, and the gravity of the suspended part of the stent was $F_{gravity}$=0.00036 N. Therefore, $F_{pull} \approx 23F_{gravity}$. At this time, the height from a center point of the farthest end cross section of the stent to a horizontal center line of a clamping section was b=1.99 mm. It was calculated according to the trigonometric function that $\theta$=3°. The stent in the above state was placed under an ultrasonic atomization nozzle for spraying. A sensofar-non-contact 3D optical profilometer was used to measure coating thicknesses of the sprayed stent, and the difference between the maximum coating thickness and the minimum coating thickness was 0.5 $\mu$m.

Embodiment 8

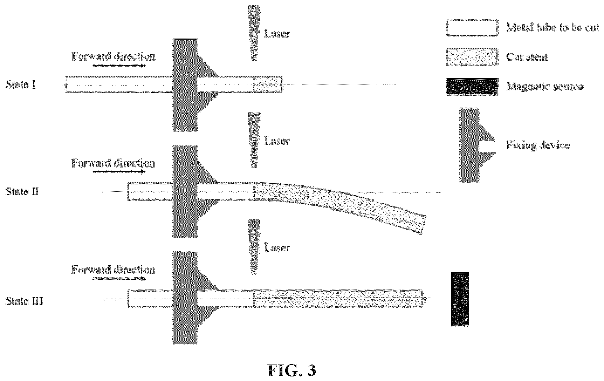

Referring to FIG. 3, a metal iron tube to be cut having an outer diameter of 1.6 mm and a length of 200 mm was clamped on a fixing device. A material was rotatably fed into a left side at a constant speed of 6 mm/s, and a right side was cut by laser into a specific patterned stent. During cutting of a long stent, for example, in state I, when pattern cutting was completely performed on only a small section of the stent, due to the hardness of the material of the metal tube, the stent was kept at certain flatness, which would not affect the energy of the laser reaching a surface of the material. When the stent was cut to state II, the cutting length of the stent reached a certain value. When the length of the suspended and hollow section of stent is relatively long, the stent at the suspended end will tilt due to the force of gravity. At this time, the inclination angle $\theta$ increases as the length of the stent that has been subjected to pattern cutting increases. As a result, the distance from a part to be cut on a surface of the stent to the laser source was different from the distance originally set for laser cutting. If the difference was larger, the laser energy reaching the surface of the material was lower. Therefore, it is possible that the stent might not be thoroughly cut or a pattern size of the stent after cutting has a relatively large deviation. In this case, a variable electromagnetic source that could have a maximum magnetic induction intensity of 1000 mT was added at the suspended end of the stent, so that the magnitude of the magnetic induction intensity might be adjusted through the magnetic source; and/or, the magnitude of the magnetic induction intensity on the suspended end of the metal tube might be adjusted by adjusting the distance between the magnetic source and the metal tube. In this embodiment, at the beginning of cutting, the magnetic source was arranged at a position that is 5 mm away from a tail end of the stent. As the metal tube moved towards the magnetic source, on the one hand, the hollow stent was gradually lengthened; and on the other hand, because the distance between the stent and the magnetic source was shortened, there is no need to adjust the magnetic source. As more and more parts of the metal tube were cut, the length of the suspended end increased, and the magnetic field might adjust the intensity of the magnetic field according to the distance/angle of deviation of the stent relative to the original center position to ensure that the angle of deviation of the stent relative to the center axis was always kept within 5°. After the cutting was completed, the height from the center point of the farthest end cross section of the stent to the horizontal center line of a clamping section was b=13.95 mm. It was calculated that $\theta$=4°<5°. Meanwhile, the cut stent was placed in a three-dimensional microscope for observation. It was obtained that the qualification rate of cutting appearance was 97% and the qualification rate of cutting size was 95%.

Comparative example 1

One end of an iron-based absorbable drug elution peripheral stent with a length of 118 mm was fixed by a clamp with a length of 59 mm (the processing outer diameter of the stent was 1.58 mm; the surface coverage of the stent was 30%; the wall thickness of the stent was 70 $\mu$m; the coverage of supporting rods on a circumferential cross section of the stent was 5%; and the mass m of the stent was 112.53 mg). The length h of a clamping-free section was 59 mm. At this time, the height from a center point of the farthest end cross section of the stent to the horizontal center line of a clamping section was b=37.92 mm. It was calculated according to the trigonometric function that $\theta$=40°. The stent in the above state was placed under an ultrasonic atomization nozzle for spraying. A sensofar-non-contact 3D optical profilometer was used to measure coating thicknesses of the sprayed stent, and the difference between the maximum coating thickness and the minimum coating thickness was 7 $\mu$m.

The foregoing descriptions are merely preferred specific implementations of the present invention, but are not intended to limit the protection scope of the present invention. Any variation or replacement easily figured out by a person skilled in the art within the technical scope disclosed in the present invention shall fall within the protection scope of the present invention. The stent in the document of the present invention is taken as an example for explanation, which does not mean that the method provided in the present invention is only applicable to the processing of the stent. Any other method for magnetically keeping a long-tubular implantable device flat and straight shall fall within the protection scope of the present invention. Therefore, the protection scope of the present invention shall be subject to the protection scope of the claims.

**Claims**

1. A method for keeping a long-tubular implanted medical device flat and straight, wherein the long-tubular implanted medical device has a fixed end and a suspended end; **characterized in that** an external force is applied to the suspended end of the long-tubular implanted medical device to ensure that the device remains flat and straight.

2. The method for keeping a long-tubular implanted medical device flat and straight according to claim 1, **characterized in that** the external force is a magnetic traction force; the magnitude of the magnetic traction force acting on the implanted medical device is $F_{pull} = \int_{x}^{x+h} \frac{B^2 S}{2 \times \mu_0} dx$ , wherein:
   $\mu_0$ is the magnetic permeability of a magnetic medium in a vacuum state; x is the distance from a magnetic source to the most epitaxial acting surface of a magnetically conductive material in the suspended end of the device; dx is the integral of the distance from the magnetic source to the acting surface of the magnetically conductive material; h is the length of the magnetically conductive material on the device in a direction perpendicular to the acting surface; $F_{pull}$ is the total traction force of the magnetic field on the device; B is the magnetic induction intensity of the magnetic field at the acting surface of the magnetically conductive material; and S is the area of the magnetic field on the acting surface of the magnetically conductive material.

3. The method for keeping a long-tubular implanted medical device flat and straight according to claim 1, **characterized in that** the magnetic source that generates the magnetic traction force is a permanent magnet or an electromagnet.

4. The method for keeping a long-tubular implanted medical device flat and straight according to claim 1, **characterized**

**in that** the value range of the distance x from the magnetic source to the most epitaxial acting surface of the magnetically conductive material in the suspended end of the long-tubular implanted medical device is 0.1 to 100 mm.

5. The method for keeping a long-tubular implanted medical device flat and straight according to claim 1, **characterized in that** the magnetic induction intensity of the magnetic field generated by the magnetic source is B ≤ 1000 mT.

6. The method for keeping a long-tubular implanted medical device flat and straight according to claim 1, **characterized in that** the length of a suspended part of the implanted medical device is 5 mm to 200 mm.

7. The method for keeping a long-tubular implanted medical device flat and straight according to claim 1, **characterized in that** the magnitude of the magnetic traction force acting on the implanted medical device is 1 to 40 times a gravity on the suspended part of the implanted medical device.

8. The method for keeping a long-tubular implanted medical device flat and straight according to claim 1, **characterized in that** the length of a device fixed at the fixed end of the implanted medical device is $L_{fixed} \leq \frac{2}{3} L_{device}$ .

9. The method for keeping a long-tubular implanted medical device flat and straight according to claim 1, **characterized in that** a fixing method for the fixed end of the implanted medical device includes clamping, physical interference fit, magnetic connection, and chemical fixation.

10. The method for keeping a long-tubular implanted medical device flat and straight according to claim 1, **characterized in that** the outer diameter of the long-tubular implantable medical device is 1.0 mm to 20.0 mm.

11. The method for keeping a long-tubular implanted medical device flat and straight according to claim 1, **characterized in that** the wall thickness of the long-tubular implanted medical device is 10 $\mu$m to 600 $\mu$m.

12. The method for keeping a long-tubular implanted medical device flat and straight according to claim 1, **characterized in that** the coverage of a cross section of the long-tubular implanted medical device is 0.1% to 35%.

13. The method for keeping a long-tubular implanted medical device flat and straight according to claim 1, **characterized in that** the surface coverage of the long-tubular implanted medical device is 5% to 60%.

14. The method for keeping a long-tubular implanted medical device flat and straight according to claim 1, **characterized in that** the implanted medical device itself has magnetic conductivity; or, a magnetically conductive patch is added onto the implanted medical device.

15. The method for keeping a long-tubular implanted medical device flat and straight according to claim 1, **characterized in that** the long-tubular implanted medical device is a raw material or semi-finished product to be processed into a stent, or a finished stent product.

**Magnetic source**

**Clamp**

**Stent**

State I

$\theta$

b

$F_{gravity}$

h

x

State II

$F_{pull}$

$\theta$

b

$F_{gravity}$

**FIG. 1**

A

A

Material with good magnetic conductivity

Material with poor magnetic conductivity

**FIG. 2**

**FIG. 3**

## INTERNATIONAL SEARCH REPORT

| International application No. |
| --- |
| **PCT/CN2022/141879** |

**A. CLASSIFICATION OF SUBJECT MATTER**

A61F2/08(2006.01)i

According to International Patent Classification (IPC) or to both national classification and IPC

**B. FIELDS SEARCHED**

Minimum documentation searched (classification system followed by classification symbols)

IPC:A61F

Documentation searched other than minimum documentation to the extent that such documents are included in the fields searched

Electronic data base consulted during the international search (name of data base and, where practicable, search terms used)

CNTXT, CNKI, 读秀, DUXIU: 支架, 管, 工件, 平直, 水平, 调节角度, 夹具, 磁, 牵引, 甩尾, 甩管; EPTXT, USTXT, WOTXT, VEN: stent, tube, workpiece, horizontal, straight, adjust angle, clamp, magnet, tract, throw, flick

**C. DOCUMENTS CONSIDERED TO BE RELEVANT**

| Category* | Citation of document, with indication, where appropriate, of the relevant passages | Relevant to claim No. |
| --- | --- | --- |
| E | CN 218190413 U (YUANXIN TECHNOLOGY (SHENZHEN) CO., LTD.) 03 January 2023 (2023-01-03)<br>description, paragraphs 3-89, and figures 1-16 | 1, 3, 9, 14-15 |
| X | CN 109773820 A (LIFETECH SCIENTIFIC (SHENZHEN) CO., LTD.) 21 May 2019 (2019-05-21)<br>description, paragraphs 5-87, and figures 1-6 | 1-15 |
| Y | CN 110756353 A (LIFETECH SCIENTIFIC (SHENZHEN) CO., LTD.) 07 February 2020 (2020-02-07)<br>description, paragraphs 6-62, and figures 1-5 | 1-15 |
| Y | CN 111037344 A (HUAZHONG UNIVERSITY OF SCIENCE AND TECHNOLOGY) 21 April 2020 (2020-04-21)<br>description, paragraphs 4-44, and figure 1 | 1-15 |
| A | CN 1535189 A (BULTMANN GMBH) 06 October 2004 (2004-10-06)<br>entire document | 1-15 |

☑ Further documents are listed in the continuation of Box C.      ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| **03 March 2023** | **10 March 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

## INTERNATIONAL SEARCH REPORT

| International application No. |
| --- |
| **PCT/CN2022/141879** |

### C.    DOCUMENTS CONSIDERED TO BE RELEVANT

| Category* | Citation of document, with indication, where appropriate, of the relevant passages | Relevant to claim No. |
| --- | --- | --- |
| A | CN 212384723 U (JINAN ACME CNC EQUIPMENT CO., LTD.) 22 January 2021 (2021-01-22)<br>entire document | 1-15 |
| A | US 2014341251 A1 (Christoph Diederichs et al.) 20 November 2014 (2014-11-20)<br>entire document | 1-15 |

Form PCT/ISA/210 (second sheet) (July 2022)

INTERNATIONAL SEARCH REPORT
Information on patent family members

International application No.

**PCT/CN2022/141879**

| Patent document cited in search report | | | Publication date (day/month/year) | Patent family member(s) | | | Publication date (day/month/year) |
|---|---|---|---|---|---|---|---|
| CN | 218190413 | U | 03 January 2023 | None | | | |
| CN | 109773820 | A | 21 May 2019 | CN | 109773820 | B | 20 September 2022 |
| CN | 110756353 | A | 07 February 2020 | None | | | |
| CN | 111037344 | A | 21 April 2020 | None | | | |
| CN | 1535189 | A | 06 October 2004 | AT | 445468 | T | 15 October 2009 |
| | | | | DE | 10135876 | A1 | 20 February 2003 |
| | | | | DE | 10135876 | C2 | 13 November 2003 |
| | | | | DE | 50213925 | D1 | 26 November 2009 |
| | | | | EP | 1409170 | A1 | 21 April 2004 |
| | | | | EP | 1409170 | B1 | 14 October 2009 |
| | | | | WO | 03009949 | A1 | 06 February 2003 |
| | | | | US | 2005022571 | A1 | 03 February 2005 |
| | | | | AU | 2002345020 | A1 | 17 February 2003 |
| | | | | DE | 20122031 | U1 | 18 March 2004 |
| CN | 212384723 | U | 22 January 2021 | None | | | |
| US | 2014341251 | A1 | 20 November 2014 | WO | 2013026542 | A1 | 28 February 2013 |
| | | | | EP | 2559995 | A1 | 20 February 2013 |
| | | | | EP | 2559995 | B1 | 31 July 2013 |

Form PCT/ISA/210 (patent family annex) (July 2022)